Europäisches Patentamt

(19) European Patent Office

Office européen des brevets

(11) Numéro de publication : **0 287 481 B1**

(12) **FASCICULE DE BREVET EUROPEEN**

(45) Date de publication du fascicule du brevet :
**11.12.91 Bulletin 91/50**

(51) Int. Cl.⁵ : **A61L 2/24, A61L 2/22**

(21) Numéro de dépôt : **88420111.2**

(22) Date de dépôt : **05.04.88**

(54) **Dispositif de nettoyage et de désinfection d'instruments médicaux et chirurgicaux.**

(30) Priorité : **13.04.87 FR 8705564**

(43) Date de publication de la demande :
**19.10.88 Bulletin 88/42**

(45) Mention de la délivrance du brevet :
**11.12.91 Bulletin 91/50**

(84) Etats contractants désignés :
**AT BE CH DE ES FR GB GR IT LI LU NL SE**

(56) Documents cités :
**CH-A- 511 030**

(73) Titulaire : **Perrot, Jean Marie Victor Aimé**
**304 rue Garibaldi**
**F-69007 Lyon (FR)**

(72) Inventeur : **Perrot, Jean Marie Victor Aimé**
**304 rue Garibaldi**
**F-69007 Lyon (FR)**

(74) Mandataire : **Maureau, Philippe et al**
**Cabinet Germain & Maureau Le Britannia -**
**Tour C 20, bld Eugène Déruelle Boîte Postale**
**3011**
**F-69392 Lyon Cédex 03 (FR)**

# Description

La présente invention a pour objet un dispositif de nettoyage et de désinfection d'instruments médicaux et chirurgicaux. Les praticiens, qu'il s'agisse de chirurgiens, de chirurgiens dentistes ou de médecins, utilisent des instruments qu'il n'est pas toujours possible de stériliser ou de désinfecter parfaitement entre deux interventions successives.

Tel est notamment le cas des pièces à mains, contre-angle, appareils à ultra-sons et vaporisateurs à trois voies utilisés en permanence par les chirurgiens dentistes.

En effet, ces instruments étant solidaires de la console de soins, il n'est pas possible de les démonter pour réaliser leur nettoyage complet entre deux interventions successives.

Or, ces instruments peuvent subir des souillures importantes à partir de microbes ou virus contenus à l'intérieur de dents gangrènées, à l'extérieur des dents dans la plaque dentaire, dans la salive ou dans le sang. Ces microbes ou virus peuvent donc être pulvérisés, sous l'action de la turbine dans la bouche du patient, sur l'instrument de base lui même, sur le miroir, sur l'écarteur de langue ou encore sur la sonde dont se sert le praticien pour protéger les téguments vis-à-vis d'un coup de fraise intempestif. Outre les risques de contamination d'un patient par un autre, il existe des risques importants de contamination du personnel médical.

De telles contaminations ont des conséquences particulièrement graves dans le cas de maladies difficiles à traiter telles que l'hépatite B ou pour lesquelles on ne dispose pas encore de traitement tel que le Syndrome Immuno Déficitaire Acquis SIDA.

Le document CH-A-511030 décrit un appareil lave-mains pour hôpitaux comportant une enceinte dans laquelle débouchent des tubulures d'amenée de liquide désinfectant, d'eau, et d'air chaud assurant les étapes de lavage et de désinfection selon un cycle programmé déclenché par actionnement d'une pédale ou par une cellule photo-électrique. L'accès à l'enceinte est rendu possible par des ouvertures obturables pour permettre un passage avec étanchéité des mains.

La présente invention vise à fournir un dispositif permettant de réaliser simplement et rapidement le nettoyage et la désinfection d'un instrument, évitant tout risque de transmission de microbes ou de virus.

A cet effet, le dispositif qu'elle concerne comprend en combinaison :

— un corps tubulaire dont une extrémité est ouverte et dont l'autre extrémité est équipée d'un réservoir de recueil de liquide, la paroi inférieure du corps tubulaire étant inclinée de haut en bas et de l'extrémité ouverte de celui-ci vers son extrémité équipée du réservoir,

— des moyens de détection de présence d'un instrument à l'intérieur du corps tubulaire, situés à proximité de l'extrémité ouverte de celui-ci,

— des moyens de diffusion sous pression d'un produit de désinfection nébulisé, actionnés par les moyens de détection lorsque ceux-ci décèlent la présence d'un instrument, et

— un dispositif d'aspiration équipant le corps tubulaire, du côté de son extrémité comportant le réservoir de recueil de liquide.

Le praticien ayant en main un instrument à nettoyer, il lui suffit d'introduire celui-ci dans le corps tubulaire du dispositif, cet instrument recevant, après détection de sa présence, une pulvérisation de fluide de désinfection. Compte tenu de la pression du fluide diffusé, ce fluide assure non seulement une désinfection de l'instrument, mais également un nettoyage de celui-ci, les souillures sous forme liquide ou sous forme de particules étant chassées sous l'effet de la pression du fluide.

Cet appareil est intéressant en raison du caractère automatique de sa commande, ce qui permet à un praticien, ne disposant que d'une main de libre, d'effectuer le nettoyage et la désinfection d'un instrument à l'issue d'une intervention et/ou préalablement à une intervention.

L'inclinaison de la paroi inférieure du corps tubulaire assure une évacuation par gravité du liquide de désinfection condensé et des souillures vers le réservoir de recueil.

Compte tenu de la présence du dispositif d'aspiration du côté du réservoir, le liquide nébulisé ne peut s'échapper hors du corps tubulaire, bien que l'extrémité avant de celui-ci demeure ouverte. Or une telle sortie de liquide nébulisé serait désagréable et même dangereuse pour le praticien. Le dispositif d'aspiration peut être solidaire du dispositif, ou être situé à distance et relié à ce dernier par une tubulure.

Selon une autre caractéristique de l'invention, les moyens de diffusion du fluide de désinfection sont constitués par une pluralité d'injecteurs, régulièrement répartis à la périphérie du corps et débouchant à l'intérieur de celui-ci, chaque injecteur comportant une chambre de mélange dans laquelle l'air est amené au centre et le liquide désinfectant est amené de façon périphérique.

L'air sous pression peut être fourni par le réseau d'air comprimé équipant toute salle d'opération et tout cabinet de chirurgiens dentistes. Les différents injecteurs sont également alimentés en liquide désinfectant à partir d'un réservoir de liquide situé à proximité du dispositif. Le nombre des injecteurs et leurs dispositions sont fonction de l'angle de diffusion de chacun d'eux, l'essentiel étant d'assurer une projection de fluide sur toute la périphérie de l'instrument.

Selon une forme d'exécution, le corps du dispositif comprend une première partie tubulaire située du côté de l'extrémité ouverte, contenant les moyens de détection de présence et de diffusion du produit et une seconde partie tubulaire qui, montée de façon amovible sur la première, est elle-même équipée de façon amovible, à son autre extrémité du réservoir de recueil.

Quoique diverses solutions puissent être envisagées, corps monolithique, intégrant ou non un réservoir, la dernière possibilité évoquée est avantageuse puisque permettant de rassembler, dans la première partie tubulaire, les différents organes nécessaires au fonctionnement du dispositif et de disposer de deux autres parties de structure très simple. Il est ainsi possible au praticien de retirer, en cours de journée, le réservoir pour vider celui-ci et de retirer en fin de journée, d'une part, le réservoir et, d'autre part, la seconde partie tubulaire en vue de réaliser leur stérilisation.

Afin de favoriser l'écoulement du liquide condensé et des salissures à l'intérieur de l'élément tubulaire, et de limiter la souillure de celui-ci, les injecteurs et éléments de détecteur de présence ne sont pas situés sur la génératrice basse du corps tubulaire.

Selon une forme d'exécution, les éléments de détection de présence de l'instrument sont situés en amont du dispositif de diffusion du liquide de nettoyage et de désinfection, dans le sens d'introduction d'un instrument dans le corps tubulaire.

Selon une autre possibilité, ce dispositif comporte, sur sa face avant, des éléments de détection de présence de la main du praticien tenant l'instrument à nettoyer.

Conformément à une forme avantageuse d'exécution, le corps tubulaire est équipé entre les moyens de diffusion du produit et la prise d'aspiration, d'un système de chicanes destinées à favoriser la condensation du produit nébulisé, et son évacuation vers le réservoir.

Avantageusement, et pour éviter de mouiller excessivement les instruments, le liquide de désinfection utilisé est non explosif, à évaporation rapide, et à action bactéricide, fongicide et virucide. A l'aide d'un tel liquide, le nettoyage et la désinfection sont réalisés en quelques secondes sous un jet de fluide à une pression inférieure à cinq bars, par exemple de l'ordre de deux bars à quatre bars.

De toute façon, l'invention sera bien comprise à l'aide de la description qui suit, en référence au dessin schématique annexé représentant, à titre d'exemple non limitatif, une forme d'exécution de ce dispositif :

Figure 1 en est une vue en perspective ;
Figure 2 en est une vue en coupe longitudinale ;

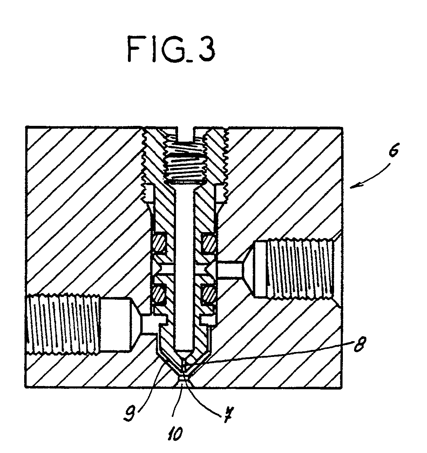

Figure 3 est une vue en coupe et à échelle agrandie d'un injecteur.

L'appareil, représenté au dessin, comprend un corps tubulaire désigné par la référence générale 2 comportant une partie avant 3 et une partie arrière 4. La partie avant, en forme de couronne, est équipée d'un dispositif de détection de présence d'un instrument constitué, dans la forme d'exécution représentée au dessin par une cellule photoélectrique ou électro-magnétique 5.

Une variante consiste à prévoir des détecteurs de présence 5a situés sur la face avant du corps, qui décèlent pour leur part la proximité de la main du praticien tenant l'instrument.

A proximité de la cellule photoélectrique 5 sont disposés une pluralité d'injecteurs 6 régulièrement répartis sur la périphérie de l'élément 3 et débouchant dans la cavité centrale de celui-ci.

Chaque injecteur 6 comprend une chambre de mélange 7 d'air et d'un liquide désinfectant dans laquelle l'air est amené par un orifice central 8 et le liquide désinfectant est amené par un orifice périphérique 9. L'air sous pression aspire le liquide qui est nébulisé, le mélange sortant dans la partie centrale du corps de l'appareil par un orifice 10.

Dans la forme d'exécution représentée au dessin, chaque injecteur est disposé radialement, quoiqu'il soit également possible d'envisager une position inclinée des injecteurs. L'air est amené à chaque injecteur par un conduit 12 relié à un réseau d'air sous pression de l'installation, tandis que le liquide désinfectant est amené par une tubulure 13 à partir d'un réservoir non représenté au dessin.

Dans la forme d'exécution représentée au dessin, la partie avant 3 du corps de l'appareil est solidaire d'un bras 14 permettant sa fixation sur une console de soins. Sur la partie 3 est fixée, par exemple par encliquetage, la partie tubulaire 4 dont le fond 4a est incliné de haut en bas et depuis l'extrémité de fixation sur la partie 3 vers son autre extrémité. Sur cette autre extrémité peut être fixé, par encliquetage, un réservoir 15 servant au recueil du fluide de nettoyage condensé et des salissures recueillies lors du nettoyage.

Il est à noter que, pour des raisons de facilité d'entretien de l'appareil, les injecteurs 6 et les dispositifs de détection de présence 5 ne font pas saillie à l'intérieur de celui-ci.

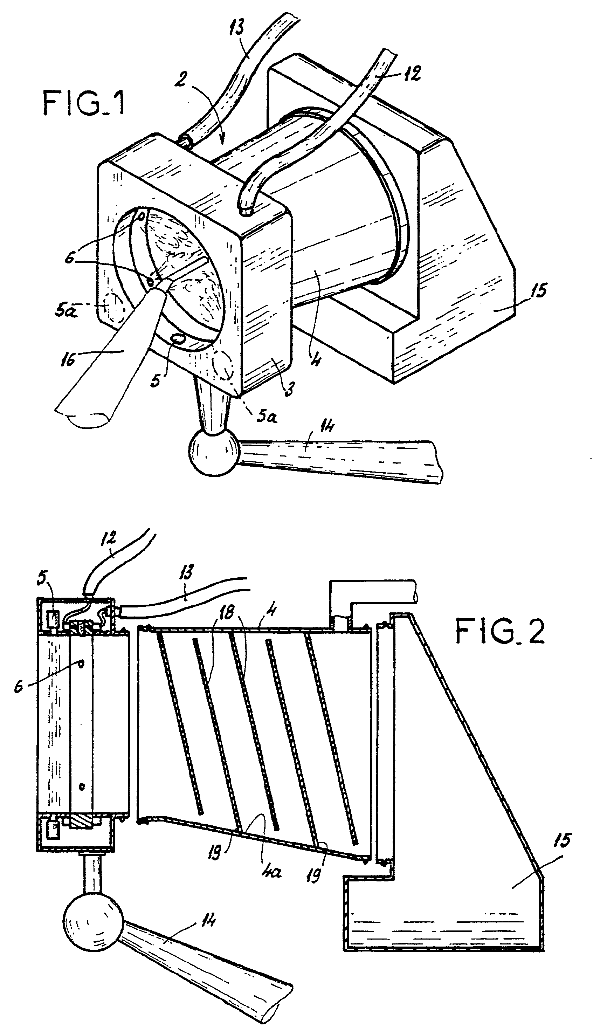

En pratique, il suffit au praticien souhaitant nettoyer et désinfecter un instrument, tel que la pièce à main 16 représentée à la figure 1, d'introduire cet instrument à l'intérieur du corps de l'appareil. Dès que la présence de cet instrument est détectée par la cellule 5, du fluide de nettoyage et de désinfection sous pression est diffusé par les injecteurs 6 sur toute la périphérie de l'instrument, pendant quelques secondes.

Au cours de cette opération, les salissures sont évacuées de l'instrument et celui-ci est désinfecté par le fluide. Le fluide condensé et les salissures sont évacués vers le réservoir 15, par gravité le long de la paroi inférieure inclinée 4a du corps tubulaire.

Selon une forme avantageuse d'exécution, le corps tubulaire 4 comporte une série de chicanes 18, ménageant toutefois un passage 19 en partie basse pour l'écoulement du liquide, ces chicanes 18 visant à favoriser la condensation du liquide nébulisé et à éviter au maximum que les particules liquides soient amenées au dispositif d'aspiration.

Comme il ressort de ce qui précède, l'invention apporte une grande amélioration à la technique existante en fournissant un dispositif de conception simple permettant de réaliser rapidement et efficacement le nettoyage et la désinfection d'instruments médicaux ou chirurgicaux, évitant ainsi les risques de contamination par l'intermédiaire de ces instruments.

**Revendications**

1. Dispositif de nettoyage et de désinfection d'instruments médicaux et chirurgicaux, caractérisé en ce qu'il comprend en combinaison :
— un corps tubulaire (3, 4) dont une extrémité est ouverte et dont l'autre extrémité est équipée d'un réservoir (15) de recueil de liquide, la paroi inférieure (4a) du corps tubulaire (4) étant inclinée de haut en bas et de l'extrémité ouverte de celui-ci vers son extrémité équipée du réservoir,
— des moyens (5) de détection de présence d'un instrument à l'intérieur du corps tubulaire, situés à proximité de l'extrémité ouverte de celui-ci,
— des moyens (6) de diffusion sous pression d'un produit de désinfection nébulisé, actionnés par les moyens de détection lorsque ceux-ci décèlent la présence d'un instrument, et
— un dispositif d'aspiration équipant le corps tubulaire (3, 4) du côté de son extrémité comportant le réservoir (15) de recueil de liquide.

2. Dispositif selon la revendication 1, caractérisé en ce que les moyens de diffusion du fluide de désinfection sont constitués par une pluralité d'injecteurs (6), régulièrement répartis à la périphérie du corps (3) et débouchant à l'intérieur de celui-ci, chaque injecteur comportant une c̶ ̶nbre de mélange (7) dans laquelle l'air est amer̶ ̶ ̶ centre et le liquide désinfectant est amené de ̶ ̶ ̶n périphérique.

3. Dispositif selon ̶ ̶ ̶e quelconque des revendications 1 et 2, caractérisé en ce que le corps du dispositif comprend une première partie tubulaire (3) située du côté de l'extrémité ouverte, contenant les moyens de détection de présence (5) et de diffusion (6) du produit et une seconde partie tubulaire (4) qui, montée de façon amovible sur la première, est elle-même équipée de façon amovible, à son autre extrémité, du réservoir de recueil (15).

4. Dispositif selon l'une quelconque des revendications 1 à 3, caractérisé en ce que les injecteurs (6) et éléments (5) de détection de présence ne sont pas situés sur la génératrice basse du corps tubulaire (3, 4).

5. Dispositif selon l'une quelconque des revendications 1 à 4, caractérisé en ce que les éléments (5) de détection de présence de l'instrument sont situés en amont du dispositif (6) de diffusion du liquide de nettoyage et de désinfection, dans le sens d'introduction d'un instrument dans le corps tubulaire.

6. Dispositif selon l'une quelconque des revendications 1 à 4, caractérisé en ce qu'il comporte, sur sa face avant, des éléments de détection de présence de la main du praticien tenant l'instrument à nettoyer.

7. Dispositif selon la revendication 1, caractérisé en ce que le corps tubulaire (3, 4) est équipé entre les moyens de diffusion du produit et la prise d'aspiration, d'un système de chicanes destinées à favoriser la condensation du produit nébulisé.

8. Dispositif selon l'une quelconque des revendications 1 à 7, caractérisé en ce que le liquide de désinfection utilisé est non explosif, à évaporation rapide, et à action bactéricide, frongicide et virucide.

**Patentansprüche**

1. Vorrichtung zum Reinigen und Desinfizieren von medizinischen und chirurgischen Instrumenten, gekennzeichnet durch die Kombination aus
einem rohrförmigen Körper (3, 4), der an einem Ende offen ist und dessen anderes Ende mit einem Behälter (5) zur Flüssigkeitsaufnahme ausgestattet ist, wobei die Innenwand (4a) des rohrförmigen Körpers (4) in Richtung von dem offenen Ende desselben zu dem mit dem Behälter ausgestatteten Ende von oben nach unten geneigt ist,
einer in der Nähe des offenen Endes des Körpers angeordneten Präsenzdetektoreinrichtung (5) zur Feststellung der Anwesenheit eines Instruments im Innern des rohrförmigen Körpers,
Mitteln (6) für das Verteilen einer zerstäubten Desinfizierflüssigkeit unter Druck, die durch die Präsenzdetektoreinrichtung aktivierbar sind, wenn diese die Anwesenheit eines Instruments feststellen, und
einer Saugvorrichtung, mit der der rohrförmige Körper (3, 4) auf der Seite desjenigen ̶ ̶des ausgestattet ist, an dem der sich der Behä̶ ̶r (15) zur Flüssigkeitsaufnahme befindet.

2. Vorrichtung nach Anspruch 1, dadurch gekennzeichnet, daß die Mittel zum Verteilen der Desinfizierflüssigkeit aus mehreren Einspritzdüsen (6) bestehen, die in regelmäßiger Anordnung am Umfang des Körpers (3) verteilt sind und im Innern des Körpers münden, wobei jede Einspritzdüse eine Mischkammer (7) aufweist, in deren Zentrum Luft eingeführt wird und in die am Umfang die Desinfizierflüssigkeit eingeführt wird.

3. Vorrichtung nach Anspruch 1 und 2, dadurch gekennzeichnet, daß der Körper der Vorrichtung einen auf der Seite des offenen Endes liegenden ersten rohrförmigen Teil (3) besitzt, der die Präsenzdetektoreinrichtung (5) und die Mittel (6) zum Verteilen der Desinfizierflüssigkeit enthält, sowie einen zweiten rohrförmigen Teil (4), der an dem ersten rohrförmigen Teil lösbar montiert ist und an dessen anderem Ende der Behälter (15) zur Flüssigkeitsaufnahme löstar befesigt ist.

4. Vorrichtung nach einem der Ansprüche 1 bis 3, dadurch gekennzeichnet, daß die Einspritzdüsen (6) und die Präsenzdetektorelemente (5) nicht auf der unteren Mantellinie des rohrförmigen Körpers (3, 4) angeordnet sind.

5. Vorrichtung nach einem der Ansprüche 1 bis 4, dadurch gekennzeichnet, daß die Präsenzdetektoreinrichtung (5) zur Feststellung der Anwesenheit eines Instruments, bezogen auf die Richtung in der ein Instrument in den rohrförmigen Körper eingeführt wird, stromaufwärts der Vorrichtung (6) zum Verteilen der Reinigungs- und Desinfizierflüssigkeit angeordnet sind.

6. Vorrichtung nach einem der Ansprüche 1 bis 4, dadurch gekennzeichnet, daß sie an ihrer Vorderseite Präsenzdetektorelemente aufweist zur Feststellung der Anwesenheit der das zu reinigende Instrument haltenden Hand des Arztes.

7. Vorrichtung nach Anspruch 1, dadurch gekennzeichnet, daß der rohrförmige Körper (3, 4) zwischen den Mitteln zum Verteilen des Produkts und dem Sauganschluß ein System von Hindernissen aufweist, die das Kondensieren des zerstäubten Produkts begünstigen

8. Vorrichtung nach einem der Ansprüche 1 bis 7, dadurch gekennzeichnet, daß die verwendete Desinfizierflüssigkeit nicht explosiv, schnell verdunstbar, bakterizid, fungizid und viruzid ist.

## Claims

1. Apparatus for cleaning and disinfecting medical and surgical instruments, characterized in that it comprises a combination of :
   — a tubular main body (3, 4) one end of which is open and the other end of which is equipped with a tank (15) for collecting liquid, the lower wall (4a) of the tubular main body (4) being tilted downwards from the open end of the latter towards the end with the tank ;
   — means (5), located near the open end of the tubular main body, for detecting the presence of an instrument inside the latter ;
   — means (6) for pressurized diffusion of a nebulised disinfectant, activated by the means of detection when the latter detect the presence of an instrument ; and
   — a suction device equipping the tubular main body (3, 4) near its end with the tank (15) for collecting liquid.

2. Apparatus according to Claim 1, characterized in that the means for diffusing the disinfectant fluid consist of a number of injectors (6), distributed at regular intervals round the periphery of the main body (3) and opening inside the latter, each injector having a mixing chamber (7) in which the air is fed in at the centre and the disinfectant liquid is fed in peripherally.

3. Apparatus according to any one of Claims 1 and 2, characterized in that the main body of the apparatus has a first tubular part (3) located near the open end, containing the means for detecting the presence (5) of and for diffusion (6) of the product and a second tubular part (4) which, set removably upon the first, is itself equipped at its other end with the collecting tank (15), the latter being removable.

4. Apparatus according to any one of Claims 1 to 3, characterized in that the injectors (6) and components (5) for detecting presence are not located on the low generatrix of the tubular main body (3, 4).

5. Apparatus according to any one of Claims 1 to 4, characterised in that the components (5) for detecting the presence of the instrument are located above the device (6) for diffusing the cleaning and disinfectant liquid, in the direction in which the instrument is inserted into the tubular main body.

6. Apparatus according to any one of Claims 1 to 4, characterized in that it has on its front face components for detecting the presence of the hand of the operator holding the instrument to be cleaned.

7. Apparatus according to Claim 1, characterized in that the tubular main body (3, 4) is equipped, between the means of diffusion of the product and the suction inlet, with a system of baffles intended to assist condensation of the nebulized product.

8. Apparatus according to any one of Claims 1 to 7, characterized in that the disinfectant liquid used is non-explosive, evaporates rapidly, and acts in a bactericidal, fungicidal and viricidal manner.

FIG_1

FIG_2

# FIG. 3